# EUROPEAN PATENT SPECIFICATION

(11) **EP 2 331 004 B1**
(45) Date of publication and mention of the grant of the patent: **07.06.2023**
(21) Application number: 09736292.5
(22) Date of filing: 22.09.2009
(51) Int. Cl.: A61C 7/08, A61C 17/22, A61H 13/00

(54) **SECTIONED MOUTHPIECE FOR ORAL CARE**
UNTERTEILTES MUNDSTÜCK FÜR DIE MUNDPFLEGE
CALE-DENTS À SECTIONS DESTINÉ À L HYGIÈNE BUCCALE

(30) Priority: 01.10.2008 US 101712 P
(43) Date of publication of application: 15.06.2011
(73) Proprietor: Koninklijke Philips N.V., 5656 AG Eindhoven (NL)
(72) Inventor: STAPELBROEK, Martinus, Bernardus, NL-5656 AE Eindhoven (NL); VEENDIJK, Arif, NL-5656 AE Eindhoven (NL)
(74) Representative: Philips Intellectual Property & Standards
(86) International application number: PCT/IB2009/054152
(87) International publication number: WO 2010/038171

(56) References cited:
- EP-A- 1 398 061
- EP-A1- 1 323 393
- WO-A-2007/128848
- US-A- 4 011 616
- US-A1- 2003 224 313
- US-A1- 2004 170 941
- US-A1- 2007 074 729

## Description

This invention relates generally to mouthpieces useful for oral care, and more specifically concerns such a mouthpiece adapted so that it can fit a large percentage of the population.

Generally, for a mouthpiece adapted for oral care, such as teeth cleaning, it is important that the mouthpiece comfortably fit the dental arch of the user. This can include just the upper or lower jaws or both, depending upon the desired function of the mouthpiece. Actual measurements have demonstrated that the dental arch can vary significantly from person to person. Thus, it has been difficult heretofore for a single mouthpiece to fit most or even a majority of the population.

Hence, it is desirable to have a mouthpiece for oral care arranged to fit at least a majority of the population, thereby eliminating the necessity of a large number of specific mouthpiece configurations.

EP 1 398 061 A2 discloses an oral appliance for placing in a mouth of a user, comprising a base member having a generally U-shaped form corresponding to the outline of a jaw of a user and defining at least one channel within which an upper or lower row of teeth of a user can be received. The base member is formed at least primarily from a material which is not user conformable or moldable in boiling water. The oral appliance further comprises a teeth engaging element which is associated with each channel and which is made of a material able to be user-conformed or user-molded to suit the individual mouth of the user. The oral appliance is manufactured by molding the U-shaped base member in a first molding step, wherein the base member is formed from a rigid plastic material, and subsequently molding a continuous layer of thermoplastic material onto the base member to form the teeth engaging element. In an embodiment, the oral appliance includes a notch or cut-out in the upper surface of an outer flange of the base member, which serves for permitting inward and outward adjustment of the arms of the U-shaped member without causing distortion of the appliance. This enables a single appliance to fit users with different arch sizes.

The oral appliance known from EP 1 398 061 A2 is especially intended for use as a guard for protecting the teeth of a user in contact sports. Further, a possibility to use the appliance for orthodontic treatment is disclosed. In the latter case, the appliance is without breathing holes and has a thinner base element, and is especially designed for myofunctional training and tooth alignment.

US 4 011 616 A discloses a vibrating toothbrush comprising a soft shoe bristled on one side and having a structural skeleton fabricated of a ductile metal. The skeleton is embedded in a covering fabricated of a soft flexible material. The shoe is capable of being formed over a human dental arch so that the bristles will be in contact with the teeth. The shoe can be detachably connected to a horseshoe-shaped base which in turn is connected to a vibrating apparatus.

US 2007/074729 A1 discloses a mandibular advancement splint against snoring and sleep apnea. The splint is made of two deformable trays designed to envelop the upper and lower arch. In order to be able to adapt to individual variations in conformation, the advancement splint includes an articulated frame having rigid and flexible elements, immersed in the thermoformable flexible material or molded around it. The frame's articulations allow the splint to fit the curve of the dental arch and to adapt to irregularities in the teeth's position.

According to the invention, a mouthpiece for oral care applications is provided, comprising:
a mouthpiece body for receiving teeth situated in one or both of the upper and lower jaws, wherein the mouthpiece body has at least one portion having a U-shaped configuration with vertical walls adapted to extend along the side surfaces of the teeth and an intermediate wall adapted to fit against the horizontal surfaces of the teeth, wherein the mouthpiece body is for cleaning of teeth, and wherein the mouthpiece body further includes brush elements on an interior surface of the mouthpiece body, and a system for moving the brush elements to provide the cleaning function for the teeth, wherein further the mouthpiece body includes at least two spaced teeth receiving sections joined by an intermediate flexible member, such that the two sections can move laterally relative to each other to accommodate various dental arches.

In comparison to the toothbrush known from US 4 011 616 A, the feature of the mouthpiece body including at least two spaced teeth receiving sections joined by an intermediate flexible member, such that the two sections can move laterally relative to each other to accommodate various dental arches constitutes a distinguishing feature.
Figure 1 is a perspective view of a mouthpiece incorporating the structure of the present invention.
Figure 2 is a top view of the mouthpiece of Figure 1.
Figure 3 is a another top view of the mouthpiece of Figures 1 and 2 showing a range of movement of sections of the mouthpiece.
Figure 4 is a simple top view of another embodiment of the mouthpiece.
Figure 5 is a simple top view of a further embodiment of the mouthpiece.
Figure 6 is a perspective view of a mouthpiece adapted to fit just the upper jaw.

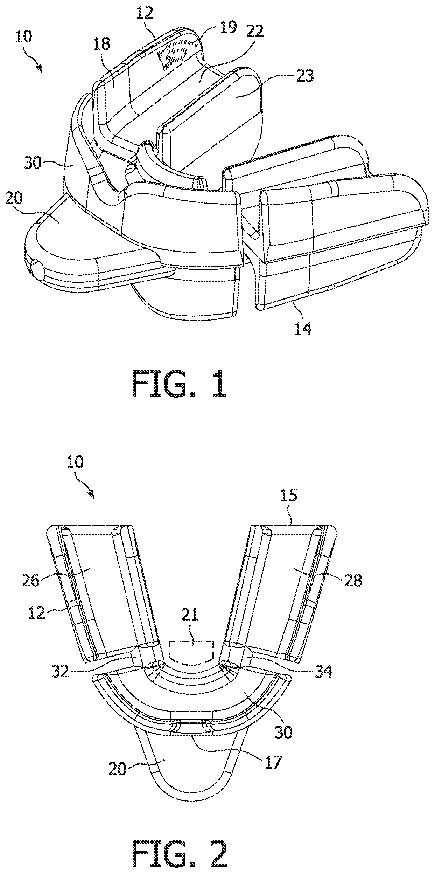

As stated above, there is a variance in dental arch configuration within the population, which typically has prevented any known mouthpiece from accommodating a substantial percentage of the population. Multiple mouthpieces having various dental arch configurations must hence be available in order to accommodate the population or a substantial portion thereof. Figures 1 and 2 show one embodiment of a mouthpiece which is configured and arranged to accommodate, i.e. operatively receive, a large number of different dental arches so that a single mouthpiece can be used by a large percentage of, if not virtually all, the population.

A mouthpiece body 10 has an upper portion 12 to receive teeth in the upper jaw and a lower portion 14 to receive the teeth in the lower jaw. The mouthpiece body 10 from a posterior end 15 to an anterior end 17 is large enough to accommodate all of the teeth in the upper and lower jaws. At the anterior end 17 is a handle element 20 by which the user can conveniently insert and remove the mouthpiece body 10 into and from the mouth.

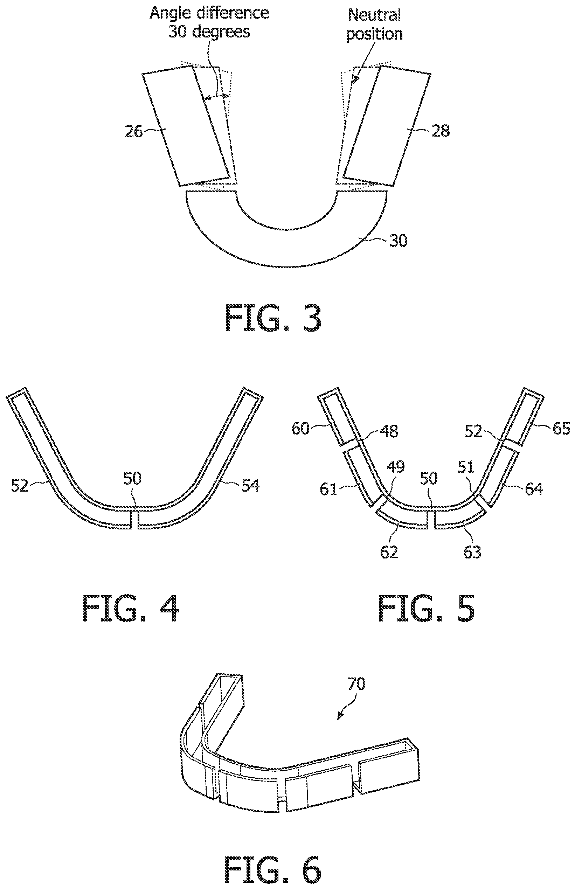

The mouthpiece body 10 can be used for a variety of oral care functions. When it is used to clean teeth, it will include bristles 19 or similar elements (shown partially in Figures 1 and 4) on the interior surfaces of the upper and/or lower portions 12 and 14, as well as a motor assembly (shown at 21) to move the bristles 19 to produce teeth cleansing. Such a brushing structure is shown and described in detail in US 9,526,597 B2. As indicated above, the upper and lower portions 12 and 14 of the mouthpiece body 10 are configured to receive teeth in such an embodiment. Typically, the upper and lower portions 12 and 14 have a U-shaped configuration, with vertical walls 18 and 23 adapted to extend along the side surfaces of the teeth, while intermediate wall 22 fits against the horizontal surfaces of the teeth.

In the embodiment of Figures 1 and 2, the mouthpiece body 10 includes three separate sections, two side sections 26 and 28 and an intermediate front section 30. The side sections 26 and 28 are joined, respectively, to the front section 30 by flexible hinges 32 and 34. In the embodiment shown, the hinges 32 and 34 are made from a silicon material, as is the remainder of the mouthpiece body 10. The hinges 32 and 34 are located at or near the interior vertical wall 23 of the mouthpiece body 10 and in cross-section are each approximately 7 mm square, and are approximately 3.5 mm long. In the embodiment shown, the distance between the two hinges 32 and 34 is approximately 25 mm, although this can vary. The arrangement of Figures 1 and 2 permits side sections 26 and 28 to move somewhat laterally relative to the front section 30 thus accommodating a variety of dental arch configurations. In the embodiment shown, the side sections 26 and 28 move through a range of approximately 30°, as shown in Figure 3, although this also can vary. The centerlines of the flexible hinges 32 and 34 are typically at an angle of approximately 37° on opposite sides of the centerline of the mouthpiece body 10 when the side sections 32 and 34 are in a neutral (relaxed) position, i.e. when the mouthpiece is out of the mouth. This angular position can vary to some extent, within the range 25° - 50°. The angular position of the flexible hinges 32 and 34, as well as its dimensions, are important in order that the mouthpiece accommodate the greatest number of dental arches within the population.

While the embodiment shown comprises silicon, hinges that are integral with the remainder of the mouthpiece body 10, *i.e.* the side and front sections 26, 28 and 30, the hinges could be of a different material than the remainder of the mouthpiece body 10 or could be like a conventional hinge with separate elements extending from the side/front sections 26, 28 and 30, rotatable about a center pin. Functionally and structurally, it is important that the two side sections 26 and 28 of the mouthpiece body 10 be readily moveable laterally relative to the front section 30, in order to accommodate different dental arch configurations.

Figure 4 shows a simple top view of another embodiment of the mouthpiece, in which a single central hinge 50 is used to connect to opposing (left and right) sections 52, 54 of the toothbrush body 10. A central hinge can also be added to the two-hinge embodiment described above in Figures 1 and 2 to produce a three-hinge arrangement. A still further embodiment is shown in Figure 5, in which a mouthpiece body 10 comprises a total of six sections 60 to 65, the sections 60 to 65 being connected to each other by intermediate flexible hinges, 48-52. In detail, the center hinge 50 connects a first set of two side sections 62 and 63, with hinges 49 and 51 connecting sections 62 and 61 and sections 63 and 64, respectively. Hinges 48 and 52 connect sections 61 and 60 and section 64 and 65, respectively.

In a variation of the embodiment of Figures 1-3, the mouthpiece could be arranged to receive just the teeth from the upper jaw or just the teeth from the lower jaw. Figure 6 shows such a mouthpiece 70 for just the teeth from the upper jaw, with a total of five sections connected by flexible hinges. As a further variation, instead of a single hinge providing flexible movement between adjacent sections which accommodate both upper and lower jaws, each section could comprise two parts, e.g. two part side sections relative to a single part front section, with each side section part accommodating one of the jaws. Each such part is separately hinged to the adjacent section. This arrangement provides further accommodation capability for different dental arches.

Accordingly, a mouthpiece for use in oral care applications has been disclosed which is able to accommodate a large percentage of different dental arches.

The oral care applications as can be accommodated with the present structure specifically include teeth cleaning and plaque removal. It should be understood that the bristles 19 included in the mouthpiece could bridge the hinges, either with larger bristles in the hinge area, or positioned on the hinges and/or the adjacent sections of the mouthpiece body 10, to maintain the functionality of cleaning around the hinge areas.

## Claims

1. A mouthpiece for oral care application, comprising:
a mouthpiece body (10) for receiving teeth situated in one or both of the upper and lower jaws, wherein the mouthpiece body (10) has at least one portion (12, 14) having a U-shaped configuration with vertical walls (18, 23) adapted to extend along the side surfaces of the teeth and an intermediate wall (22) adapted to fit against the horizontal surfaces of the teeth, wherein the mouthpiece body (10) is for cleaning of teeth, and wherein the mouthpiece body (10) further includes brush elements (19) on an interior surface of the mouthpiece body (10), and a system (21) for moving the brush elements (19) to provide the cleaning function for the teeth,
**characterized in that** the mouthpiece body (10) includes at least two spaced teeth receiving sections (26, 28, 30; 52, 54; 60-65) joined by an intermediate flexible member (32, 34; 50; 48-52), such that the two sections (26, 28, 30; 52, 54; 60-65) can move laterally relative to each other to accommodate various dental arches.

2. The mouthpiece of claim 1, wherein the mouthpiece body (10) comprises a total of three separate spaced teeth receiving sections (26, 28, 30), including a center section (30) and two side sections (26, 28), and two intermediate flexible members (32, 34) which connect, respectively, the side sections (26, 28) to the center section (30), and wherein the two side sections (26, 28) have centerlines which are located within a range of 25° to 50° on opposite sides of a centerline of the mouthpiece body (10) when the side sections (26, 28) are at rest outside of the mouth.

3. The mouthpiece of claim 1, wherein the mouthpiece body (10) comprises two teeth receiving sections (52, 54) and a flexible member (50) joining the two teeth receiving sections (52, 54), the flexible member (50) being located approximately at a centerline of the mouthpiece body (10).

4. The mouthpiece of claim 1, wherein the mouthpiece body (10) includes a total of six teeth receiving sections (60-65), each of the teeth receiving sections (60-65) joined by a flexible member (48-52) to an adjacent teeth receiving section (60-65).

5. The mouthpiece of claim 1, wherein the mouthpiece body (10) is adapted to receive only teeth from the upper jaw of a user.

6. The mouthpiece of claim 1, wherein the mouthpiece body (10) is adapted to receive only teeth from the lower jaw of a user.

7. The mouthpiece of claim 2, wherein the mouthpiece body (10) is adapted to receive teeth from both the upper and lower jaws of a user.

8. The mouthpiece of claim 1, wherein the mouthpiece body (10) and the flexible members (32, 34; 50; 48-52) comprise a silicon material.

9. The mouthpiece of claim 1, wherein the flexible members (32, 34; 50; 48-52) are positioned at an interior vertical surface (23) of the teeth receiving sections (26, 28, 30; 52, 54; 60-65).

10. The mouthpiece of claim 1, wherein the flexible members (32, 34; 50; 48-52) are arranged such that the teeth receiving sections (26, 28, 30; 52, 54; 60-65) are movable through an angle of approximately 30°.

11. The mouthpiece of claim 1, wherein the brush elements (19) bridge the flexible members (32, 34; 50; 48-52) to provide complete cleaning of the teeth.

## Patentansprüche

1. Mundstück zur Anwendung in der Mundpflege, umfassend: einen Mundstückkörper (10) zum Aufnehmen von Zähnen, die sich in einem oder beiden von dem Ober- und Unterkiefer befinden, wobei der Mundstückkörper (10) mindestens einen Abschnitt (12, 14) aufweist, der eine U-förmige Konfiguration mit vertikalen Wänden (18, 23), die angepasst sind, um sich entlang der Seitenflächen der Zähne zu erstrecken, und eine Zwischenwand (22), die angepasst ist, um an den horizontalen Flächen der Zähne anzuliegen, aufweist wobei der Mundstückkörper (10) zum Reinigen von Zähnen dient, und wobei der Mundstückkörper (10) ferner Bürstenelemente (19) an einer Innenfläche des Mundstückkörpers (10) und ein System (21) zum Bewegen der Bürstenelemente (19) beinhaltet, um die Reinigungsfunktion für die Zähne bereitzustellen, **dadurch gekennzeichnet, dass** der Mundstückkörper (10) mindestens zwei beabstandete Zahnaufnahmeabschnitte (26, 28, 30; 52, 54; 60-65) beinhaltet, die durch ein flexibles Zwischenelement (32, 34; 50; 48-52) verbunden sind, sodass sich die zwei Abschnitte (26, 28, 30; 52, 54; 60-65) seitlich in Bezug aufeinander bewegen können, um sich verschiedenen Zahnbögen anzupassen.

2. Mundstück nach Anspruch 1, wobei der Mundstückkörper (10) insgesamt drei separate, beabstandete Zahnaufnahmeabschnitte (26, 28, 30), die einen mittleren Abschnitt (30) und zwei seitliche Abschnitte (26, 28) beinhalten, und zwei flexible Zwischenelemente (32, 34), die jeweils die seitlichen Abschnitte (26, 28) mit dem mittleren Abschnitt (30) verbinden, umfasst und wobei die zwei seitlichen Abschnitte (26, 28) Mittellinien aufweisen, die sich innerhalb eines Bereichs von 25° bis 50° auf gegenüberliegenden Seiten einer Mittellinie des Mundstückkörpers (10) befinden, wenn die seitlichen Abschnitte (26, 28) außerhalb des Munds ruhen.

3. Mundstück nach Anspruch 1, wobei der Mundstückkörper (10) zwei Zahnaufnahmeabschnitte (52, 54) und ein flexibles Element (50) umfasst, das die zwei Zahnaufnahmeabschnitte (52, 54) verbindet, wobei sich das flexible Element (50) ungefähr an einer Mittellinie des Mundstückkörpers (10) befindet.

4. Mundstück nach Anspruch 1, wobei der Mundstückkörper (10) insgesamt sechs Zahnaufnahmeabschnitte (60-65) beinhaltet, wobei jeder der Zahnaufnahmeabschnitte (60-65) durch ein flexibles Element (48-52) mit einem benachbarten Zahnaufnahmeabschnitt (60-65) verbunden ist.

5. Mundstück nach Anspruch 1, wobei der Mundstückkörper (10) angepasst ist, um nur Zähne des Oberkiefers eines Benutzers aufzunehmen.

6. Mundstück nach Anspruch 1, wobei der Mundstückkörper (10) angepasst ist, um nur Zähne des Unterkiefers eines Benutzers aufzunehmen.

7. Mundstück nach Anspruch 2, wobei der Mundstückkörper (10) angepasst ist, um Zähne sowohl des Ober- als auch des Unterkiefers eines Benutzers aufzunehmen.

8. Mundstück nach Anspruch 1, wobei der Mundstückkörper (10) und die flexiblen Elemente (32, 34; 50; 48-52) ein Silikonmaterial umfassen.

9. Mundstück nach Anspruch 1, wobei die flexiblen Elemente (32, 34; 50; 48-52) an einer inneren vertikalen Oberfläche (23) der Zahnaufnahmeabschnitte (26, 28, 30; 52, 54; 60-65) positioniert sind.

10. Mundstück nach Anspruch 1, wobei die flexiblen Elemente (32, 34; 50; 48-52) angeordnet sind, sodass die Zahnaufnahmeabschnitte (26, 28, 30; 52, 54; 60-65) um einen Winkel von etwa 30° beweglich sind.

11. Mundstück nach Anspruch 1, wobei die Bürstenelemente (19) die flexiblen Elemente (32, 34; 50; 48-52) überbrücken, um eine vollständige Reinigung der Zähne bereitzustellen.

## Revendications

1. Embout buccal pour application de soins buccodentaires, comprenant:
un corps d'embout buccal (10) destiné à recevoir des dents situées dans l'une ou les deux mâchoires supérieures et inférieures, dans lequel le corps d'embout buccal (10) a au moins une partie (12, 14) ayant une configuration en forme de U avec des parois verticales (18, 23) adaptées pour s'étendre le long des surfaces latérales des dents et une paroi intermédiaire (22) adaptée pour s'adapter contre les surfaces horizontales des dents, dans lequel le corps de l'embout buccal (10) est destiné au nettoyage des dents, et dans lequel le corps de l'embout buccal (10) comprend en outre des éléments de brosse (19) sur une surface intérieure du corps de l'embout buccal (10), et un système (21) pour déplacer les éléments de brosse (19) afin d'assurer la fonction de nettoyage des dents, **caractérisé par le fait que** le corps de l'embout buccal (10) comprend au moins deux sections espacées recevant des dents (26, 28, 30; 52, 54; 60-65) reliées par un élément intermédiaire flexible (32, 34; 50; 48-52), de sorte que les deux sections (26, 28, 30; 52, 54; 60-65) peuvent se déplacer latéralement l'une par rapport à l'autre afin de s'adapter à diverses arcades dentaires.

2. Embout buccal selon la revendication 1, dans lequel le corps de l'embout buccal (10) comprend un total de trois sections de réception de dents séparées et espacées (26, 28, 30), y compris une section centrale (30) et deux sections latérales (26, 28), et deux éléments flexibles intermédiaires (32, 34) qui relient, respectivement, les sections latérales (26, 28) à la section centrale (30), et dans lequel les deux sections latérales (26, 28) ont des lignes médianes qui sont situées dans une plage de 25° à 50° sur les côtés opposés d'une ligne médiane du corps de l'embout buccal (10) lorsque les sections latérales (26, 28) sont au repos à l'extérieur de la bouche.

3. Embout buccal selon la revendication 1, dans lequel le corps de l'embout buccal (10) comprend deux sections de réception des dents (52, 54) et un élément flexible (50) reliant les deux sections de réception des dents (52, 54), l'élément flexible (50) étant situé approximativement au niveau d'une ligne médiane du corps de l'embout buccal (10).

4. Embout buccal selon la revendication 1, dans lequel le corps de l'embout buccal (10) comprend un total de six sections de réception des dents (60-65), chacune des sections de réception des dents (60-65) étant reliée par un élément flexible (48-52) à une section de réception des dents adjacente (60-65).

5. Embout buccal selon la revendication 1, dans lequel le corps de l'embout buccal (10) est adapté pour recevoir uniquement les dents de la mâchoire supérieure d'un utilisateur.

6. Embout buccal selon la revendication 1, dans lequel le corps de l'embout buccal (10) est adapté pour recevoir uniquement les dents de la mâchoire inférieure d'un utilisateur.

7. Embout buccal selon la revendication 2, dans lequel le corps de l'embout buccal (10) est adapté pour recevoir les dents des mâchoires supérieure et inférieure d'un utilisateur.

8. Embout buccal selon la revendication 1, dans lequel le corps de l'embout buccal (10) et les éléments flexibles (32, 34; 50; 48-52) sont constitués d'un matériau en silicone.

9. Embout buccal selon la revendication 1, dans lequel les éléments flexibles (32, 34; 50; 48-52) sont positionnés sur une surface verticale intérieure (23) des sections recevant les dents (26, 28, 30; 52, 54; 60-65).

10. Embout buccal selon la revendication 1, dans lequel les éléments flexibles (32, 34; 50; 48-52) sont disposés de manière à ce que les sections recevant les dents (26, 28, 30; 52, 54; 60-65) puissent être déplacées d'un angle d'environ 30°.

11. Embout buccal selon la revendication 1, dans lequel les éléments de brosse (19) relient les Eléments flexibles (32, 34; 50; 48-52) pour assurer un nettoyage complet des dents.
